# EUROPEAN PATENT APPLICATION

(11) **EP 3 257 846 A1**
(43) Date of publication of application: **20.12.2017**
(21) Application number: 16181566.7
(22) Date of filing: 27.07.2016
(51) Int. Cl.: C07D 307/89, C07C 37/11, C07C 39/18, C07C 51/367, C07C 63/64

(54) **METHOD FOR FORMING POLYALKYNYL-ARENES**

(30) Priority: 16.06.2016 US 201615183840
(71) Applicant: Dow Global Technologies LLC, Midland, MI 48674 (US)
(72) Inventor: HANLEY, Patrick S., Midland, MI Michigan 48674 (US); JANSMA, Matthew J., Midland, MI Michigan 48674 (US); GILMORE, Christopher, Marlborough, MA Massachusetts 01752 (US)
(74) Representative: Houghton, Mark Phillip

(57) **Abstract**

A method of coupling an aromatic compound having a two or more fluorosulfonate substituents to an alkyne is useful in the preparation of polyalkynyl-substituted arene compounds.

## Description

The present invention relates to forming certain substituted-aromatic compounds and more particularly to forming polyalkynyl-arenes.

Sonogashira coupling is a valuable synthetic method for coupling an aromatic compound to an alkyne, thereby forming a new carbon-carbon bond between the aromatic compound and the alkyne. One common Sonogashira coupling reaction uses a halo-substituted aromatic compound as a starting material. Another common coupling reaction uses a hydroxyl-substituted aromatic compound.

It is known that triflates, having the formula F₃CSO₃-, may be used in the place of the halides in Sonogashira coupling reactions, however the expense of triflic anhydride ((CF₃SO₂)₂0) has limited the use of triflates in Sonogashira couplings to the production of fine chemicals. Further, the atom economy of triflic anhydride is low since half of the molecule is expended as monomeric triflate anion (CF₃SO₃⁻) following functionalization of a phenolic starting material. In some instances Sonogashira coupling reactions involving triflates exhibit sensitivity to water under basic conditions.

Aryl methanesulfonates are also suitable for Sonogashira coupling reactions, but have the drawback of requiring expensive palladium catalysts. Another drawback of using aryl methanesulfonates is low atom economy.

When performing a Sonogashira coupling using either a triflate or methanesulfonate moiety, it is common to perform the reaction in two steps, a first step comprising replacing a hydroxyl group on the aromatic compound with the triflate or the methanesulfonate, and a second step comprising coupling the aromatic compound with the alkyne. A separation step is generally required between the first and second steps.

Polyalkynyl-substituted arenes are useful as monomers in Diels-Alder polymerizations to form polyphenylene polymers. U.S. Patent Application No. 62/206,343, filed on August 18, 2015 and assigned to the same assignee as this application, discloses an improved method of forming aromatic alkynes using Sonogashira coupling. However, this application fails to disclose the formation of polyalkynyl-substituted arenes. A method of forming polyalkynyl-substituted arenes using Sonogashira coupling without the use of triflates or methanesulfonates is desired.

The present invention provides a method of forming a polyalkynyl-substituted arene compound comprising: (a) reacting a polyhydroxy-substituted aromatic compound with a fluorosulfonating agent to form a poly(fluorosulfonate)-substituted aromatic compound; and (b) reacting the poly(fluorosulfonate)-substituted aromatic compound with an alkyne in the presence of a catalyst under conditions effective to couple the alkyne to the poly(fluorosulfonate)-substituted aromatic compound and form the polyalkynyl-substituted arene compound; wherein the catalyst comprises one or more group 10 atoms or a copper complex.

The present invention also provides a method of forming a polyalkynyl-substituted arene compound comprising: (a) providing an aromatic compound having two or more fluorosulfonate substituents; and (b) reacting the aromatic compound having two or more fluorosulfonate substituents with an alkyne in a reaction mixture under conditions effective to couple the alkyne to the aromatic compound and form the polyalkynyl-substituted arene compound, the reaction mixture comprising a catalyst comprising one or more group 10 atoms or a copper complex.

As used throughout this specification, the following abbreviations shall have the following meanings, unless the context clearly indicates otherwise: °C = degree Celsius; g = gram; mg = milligram; mmol = millimole; L = liter; mL = milliliter; mmHg = millimeters of mercury; Pa = pascal; 1 mmHg = 133.3 Pa; min. = minute; hr. = hour; DI = deionized; Da = Dalton; and NMR = nuclear magnetic resonance. Unless otherwise specified, all amounts are percent by weight ("wt%") and all ratios are molar ratios. All numerical ranges are inclusive and combinable in any order, except where it is clear that such numerical ranges are constrained to add up to 100%. The articles "a", "an" and "the" refer to the singular and the plural. When an element is referred to as being "disposed on" another element, it can be directly on the other element or intervening elements may be present therebetween. In contrast, when an element is referred to as being "disposed directly on" another element, there are no intervening elements present.

"Halo" refers to fluoro, chloro, bromo, and iodo. The term "alkyl" refers to an alkane radical, and includes alkane monoradicals, diradicals (alkylene), and higher-radicals. "Alkyl" refers to linear, branched and cyclic alkyl unless otherwise specified. The term "heteroalkyl" refers to an alkyl group with one or more heteroatoms, such as nitrogen, oxygen, sulfur, phosphorus, replacing one or more carbon atoms within the radical, for example, as in an ether or a thioether. If no number of carbons is indicated for any alkyl or heteroalkyl, then 1-12 carbons are contemplated. The term "alkenyl" refers to an alkene radical, and includes alkene monoradicals, diradicals (alkenylene), and higher-radicals. "Alkenyl" refers to linear, branched and cyclic alkenyl unless otherwise specified. The term "alkynyl" refers to an alkyne radical, and includes alkyne monoradicals, diradicals, and higher-radicals. "Alkynyl" refers to linear and branched alkynyl. If no number of carbons is indicated for any alkenyl or alkynyl, then 2-12 carbons are contemplated.

The terms "arene" and "aromatic compound" are used interchangeably. As used herein, "aromatic compound" refers to a compound having one or more aryl moieties. An "aryl" moiety refers to any functional group or substituent derived from an aromatic ring. Also as used herein, the terms "aryl" and "aromatic" include "heteroaryl" and "heteroaromatic", respectively. Aryl moieties contain 4n+2 pi electrons, where n is an integer. "Heteroaryl" and "heteroaromatic" refer to any functional group or substituent derived from an aromatic ring having at least one heteroatom chosen from nitrogen, oxygen, and sulfur.

The present invention provides a method of forming a polyalkynyl-substituted arene compound comprising: (a) reacting a polyhydroxy-substituted aromatic compound with a fluorosulfonating agent to form a poly(fluorosulfonate)-substituted aromatic compound; and (b) reacting the poly(fluorosulfonate)-substituted aromatic compound with an alkyne in the presence of a catalyst under conditions effective to couple the alkyne to the poly(fluorosulfonate)-substituted aromatic compound and form the polyalkynyl-substituted arene compound; wherein the catalyst comprises one or more group 10 atoms or a copper complex. Aromatic compounds useful in the present invention have two or more hydroxy substituents on the aryl moiety, that is, two or more of the hydrogens on the aryl moiety are replaced by hydroxyls. The hydroxyl substituents may be on the same aromatic ring or on different aromatic rings of the aryl moiety. Suitable aryl moieties are any individual aromatic rings or any fused ring systems, or any combination thereof. Such fused ring systems may be composed of 2 or more, preferably from 2 to 8, fused rings, where at least one of the fused rings is aromatic. It is preferred that 2 or more of the fused rings are aromatic. Suitable fused ring systems may be composed only of fused aromatic rings, or, alternatively, may be composed of one or more aromatic rings fused with one or more non-aromatic rings. Suitable aryl moieties have from 6 to 50 aromatic ring atoms. Exemplary aryl moieties having individual (that is, non-fused) aromatic rings include, without limitation, those derived from benzene, biphenyl, terphenyl, triphenylbenzene, hexaphenylbenzene, pyridine, bipyridine, pyrimidine, pyridazine, pyrrole, imidazole, triazine, triazole, furan, oxazole, thiazole, and thiophene. Exemplary aryl moieties having fused aromatic rings include, without limitation, those derived from fluorene, 9,9-diphenylfluorene, 9,9-dinaphthylfluorene, carbazole, dibenzofuran, dibenzothiophene, naphthalene, acenaphthalene, binaphthalene, phenanthracene, anthracene, pyrene, indole, isoindole, triphenylene, tetracene, tetraphene, pentacene, perylene, coronene, quinoline, isoquinoline, phthalic anhydride, pyromellitic dianhydride, benzimidazole, benzotriazole, benzofuran, benzothiophene, benzoxaxole, and benzothiazole. It is preferred that the aryl moieties are not heteroaryl. Preferred are aryl moieties derived from benzene, biphenyl, terphenyl, triphenylbenzene, hexaphenylbenzene, pyridine, bipyridine, fluorene, 9,9-diphenylfluorene, 9,9-dinaphthylfluorene, carbazole, naphthalene, acenaphthalene, binaphthalene, phenanthracene, anthracene, pyrene, triphenylene, tetracene, tetraphene, pentacene, perylene, coronene, phthalic anhydride, and pyromellitic dianhydride. The aryl moiety of the aromatic compound, in addition to having two or more hydroxyl substituents, may optionally have one or more additional substituents. Exemplary additional substituents include, without limitation, boron-containing groups, alkyl, haloalkyl, halogen, C₁₋₁₂ alkoxy, C₁₋₁₂ mercaptoalkyl, C₆₋₂₀ aryloxy, dialkylphosphino, di-C₆₋₂₀ arylphosphino, dialkylphosphono, di-C₆₋₂₀ arylphosphono, C₂-C₈ alkene, tri-C₁₋₁₂ alkylsilyl, and solubility enhancing moieties. Preferably, the polyhydroxy-substituted aromatic compound also has one or more solubility enhancing moieties. Exemplary solubility enhancing moieties include, but are not limited to, hydroxyl, hydroxyalkyl (having 1 to 12 carbon atoms), thioalkyl (having 1 to 12 carbon atoms), mercaptoalkyl (having 1 to 12 carbon atoms), carboxylic acid, carboxylic acid esters (having 2 to 30 carbon atoms), amines (having 0 to 60 carbon atoms), amides (having 1 to 60 carbon atoms), ammonium salts (having 0 to 30 carbon atoms), nitro, thiol, sulfonamides (having 0 to 30 carbon atoms), cyano, sulfonic acid, and sulfonyl esters (having 1 to 30 carbon atoms).

Preferably, the polyhydroxy-substituted aromatic compound useful in the present invention has the structure (1): 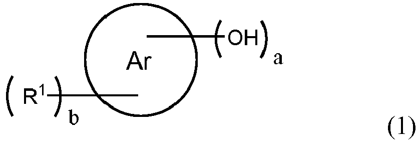 wherein Ar represents an aryl moiety having from 6 to 50 aromatic ring atoms; each R¹ is a solubility enhancing moiety; a is an integer from 2 to 6; and b is an integer from 0 to 4. Preferably, Ar is chosen from benzene, biphenyl, terphenyl, triphenylbenzene, hexaphenylbenzene, pyridine, bipyridine, pyrimidine, pyridazine, pyrrole, imidazole, triazine, triazole, furan, oxazole, thiazole, thiophene, fluorene, 9,9-diphenylfluorene, 9,9-dinaphthylfluorene, carbazole, dibenzofuran, dibenzothiophene, naphthalene, acenaphthalene, binaphthalene, phenanthracene, anthracene, pyrene, indole, isoindole, triphenylene, tetracene, tetraphene, pentacene, perylene, coronene, quinoline, isoquinoline, phthalic anhydride, pyromellitic dianhydride, benzimidazole, benzotriazole, benzofuran, benzothiophene, benzoxaxole, and benzothiazole. It is preferred that Ar is chosen from benzene, biphenyl, terphenyl, triphenylbenzene, hexaphenylbenzene, pyridine, bipyridine, fluorene, 9,9-diphenylfluorene, 9,9-dinaphthylfluorene, carbazole, naphthalene, acenaphthalene, binaphthalene, phenanthracene, anthracene, pyrene, triphenylene, tetracene, tetraphene, pentacene, perylene, coronene, phthalic anhydride, and pyromellitic dianhydride. Preferred solubility enhancing moieties of R¹ include, but are not limited to, independently chosen from -OH, C₁₋₁₂ hydroxyalkyl, -C(=O)OR³, -C(=O)N(R⁴)₂, -O-C(=O)R⁵, -NR⁴C(=O)R⁶, -NO₂;-S(=O)₂-OR⁷, -O-S(=O)₂-R⁸, -NR⁴-S(=O)₂-R⁶, S(=O)₂-N(R⁴)₂, and -Si(Z)₃; wherein R³ = H, C₁₋₁₂ alkyl, C₁₋₁₂ hydroxyalkyl, C₁₋₁₂ aminoalkyl, or C₆₋₃₀ aryl; each R⁴ is independently H, C₆₋₃₀ aryl, or C₁₋₁₂ alkyl; each R⁵ is independently chosen from H, C₁₋₁₂ alkyl, C₁₋₁₂ hydroxyalkyl, C₆₋₃₀ aryl, -O(C₁₋₁₂ alkyl), -O(C₆₋₃₀ aryl) and -N(R⁴)₂; R⁶ = H, C₁₋₁₂ alkyl, C₁₋₁₂ hydroxyalkyl, C₆₋₃₀ aryl, -O(C₁₋₁₂ alkyl), or -NH(C₁₋₁₂ alkyl); R⁷ = H, C₁₋₁₂ alkyl, or C₆₋₃₀ aryl; R⁸ = C₆₋₃₀ aryl, C₁₋₁₂ alkyl, and halo C₁₋₁₂ alkyl; each Z is independently chosen from C₁₋₁₂ alkyl, C₁₋₁₂ hydroxyalkyl, C₁₋₁₂ alkoxy, C₁₋₁₂ alkanecarboxylate, -N(R⁴)₂, or -OSi(Y)₃; and each Y is chosen from C₁₋₁₂ alkyl, C₁₋₁₂ hydroxyalkyl, C₁₋₁₂ alkoxy, or C₁₋₁₂ alkanecarboxylate. It is preferred that each R¹ is independently chosen from -OH, C₁₋₄ hydroxyalkyl, -C(=O)OR³, -C(=O)N(R⁴)₂, -O-C(=O)R⁵, -S(=O)₂-OR⁶, S(=O)₂-N(R⁴)₂, and -Si(Z)₃, more preferably from -OH, C₁₋₄ hydroxyalkyl, -C(=O)OR³ and -C(=O)N(R⁴)₂, and yet more preferably -OH and -C(=O)OR³. Preferably, R³ is H, C₁₋₆ alkyl, C₁₋₆ hydroxyalkyl, C₁₋₆ aminoalkyl, or C₆₋₃₀ aryl, more preferably H, C₁₋₄ alkyl, or C₁₋₆ hydroxyalkyl, and even more preferably H. R⁴ is preferably H, C₆₋₃₀ aryl, or C₁₋₆ alkyl, and more preferably H or C₁₋₄ alkyl. It is preferred that R⁵ is C₁₋₆ alkyl, C₁₋₆ hydroxyalkyl, C₆₋₃₀ aryl, -O(C₁₋₁₀ alkyl), or-N(R⁴)₂, and more preferably C₁₋₆ alkyl, C₁₋₆ hydroxyalkyl, C₆₋₂₀ aryl, -O(C₁₋₆ alkyl), or-N(R⁴)₂. R⁶ is preferably H, C₁₋₁₀ alkyl, C₁₋₆ hydroxyalkyl, C₆₋₂₀ aryl, -O(C₁₋₁₀ alkyl), or-N(R⁴)₂, and more preferably H, C₁₋₆ alkyl, -O(C₁₋₆ alkyl), or -N(R⁴)₂. R⁷ is preferably H, C₁₋₆ alkyl, or C₆₋₂₀ aryl, more preferably H or C₁₋₄ alkyl, and even more preferably H. It is preferred that R⁸ is C₆₋₂₀ aryl, C₁₋₁₀ alkyl, and C₁₋₁₀ fluoroalkyl, and more preferably phenyl, tolyl, methyl, and trifluoromethyl. Preferably, a = 2 to 4, and more preferably 2 to 3. It is preferred that b = 1 to 3, and more preferably b = 1 or 2. More preferably, a = 2 to 4 and b = 1 to 3, and yet more preferably a = 2 to 3 and b = 1 to 3. It will be apparent to those skilled in the art that any reactive functional groups, such as hydroxyl and carboxylic acid, in R¹ may be protected. Such hydroxyl and carboxylic acid protecting groups are well known to those skilled in the art. Suitable polyhydroxy-substituted aromatic compounds are commercially available, such as from Sigma-Aldrich, or may be prepared by procedures known in the art.

The polyhydroxy-substituted aromatic compound is reacted with a fluorosulfonating agent to form a poly(fluorosulfonate)-substituted aromatic compound. Any suitable fluorosulfonating agent may be used, and is preferably sulfuryl fluoride (SO₂F₂). Preferably, the polyhydroxy-substituted aromatic compound is reacted with the fluorosulfonating agent in the presence of a base in a suitable solvent. Optionally, a phase-transfer catalyst is used in combination with the base. In another optional embodiment, the base is used in the presence of water. In yet another instance, the base is used in the presence of an organic solvent. In still another instance, the base is used in the presence of one or more of a phase-transfer catalyst, water or an organic solvent. Optionally, a surfactant is used in the reaction mixture. Typically, the polyhydroxy-substituted aromatic compound is combined with the optional base in a suitable solvent, followed by addition of the fluorosulfonating agent. When sulfuryl fluoride is used as the fluorosulfonating agent, it is preferably used as a gas, but it may, alternatively, be used at a sufficiently low temperature such that the sulfuryl fluoride is in a liquid state.

While any suitable base compatible with the fluorosulfonating agent may be used, it is preferred that the base is selected to also be compatible with the catalyst used in the subsequent alkyne coupling step. Preferred bases include, but are not limited to, carbonate salts, phosphate salts, acetate salts and carboxylic acid salts. Inorganic bases may suitably be used in the reaction mixture. Examples of carbonate salts include, but are not limited to, lithium carbonate, sodium carbonate, potassium carbonate, rubidium carbonate, cesium carbonate, ammonium carbonate, substituted ammonium carbonates, and the corresponding hydrogen carbonate salts. Examples of phosphate salts include, without limitation, lithium phosphate, sodium phosphate, potassium phosphate, rubidium phosphate, cesium phosphate, ammonium phosphate, substituted ammonium phosphates, and the corresponding hydrogen phosphate salts of the foregoing. Examples of acetate salts include, but are not limited to, lithium acetate, sodium acetate, potassium acetate, rubidium acetate, cesium acetate, ammonium acetate, and substituted ammonium acetates.

Other suitable bases for use in the fluorosulfonating step include, but are not limited to, salts of formate, fluoroacetate, and propionate anions with lithium, sodium, potassium, rubidium, cesium, ammonium, and substituted ammonium cations; bis(silyl)amide salts such as the lithium, sodium, and potassium salts of bis(trimethylsilyl)amide; alkoxide salts such as the lithium, sodium, and potassium salts of tert-butoxide; metal fluorides, such as sodium fluoride, potassium fluoride, cesium fluoride, silver fluoride, tetrabutylammonium fluoride, ammonium fluoride, and triethyl ammonium fluoride; metal hydroxides; and non-nucleophilic organic amines such as hindered amines and heteroarenes. The term "metal hydroxides" used herein includes metal polyhydroxides such as metal dihydroxides and metal trihydroxides. Exemplary metal hydroxides include, without limitation, lithium hydroxide, sodium hydroxide, potassium hydroxide, magnesium dihydroxide, calcium dihydroxide, strontium dihydroxide, barium dihydroxide, aluminum trihydroxide, gallium trihydroxide, indium trihydroxide, and thallium trihydroxide. Exemplary non-nucleophilic organic amines include, but are not limited to, triethylamine, pyridine, morpholine, 2,6-lutidine, N,N-dicyclohexylmethylamine, diisopropylamine, N,N-diisopropylethylamine, 1,4-diazabicyclo[2.2.2]octane (DABCO), 1,5-diazabicyclo[4.3.0]non-5-ene (DBN), 1,8-diazabicyclo[5.4.0]undec-7-ene (DBU), and 1,8-bis(dimethylamino)naphthalene.

Preferably, at least one equivalent of base is present for each equivalent of fluorosulfonate. In some embodiments, no more than 10 equivalents of base are present for each equivalent of fluorosulfonate. In some embodiments, at least 2 equivalents of base are present for each equivalent of fluorosulfonate. In some embodiments, no more than 6 equivalents of base are present for each equivalent of fluorosulfonate.

The solvent in the fluorosulfonating reaction mixture is selected such that it is suitable for use with the reactants, the base, and also preferably suitable for use with the reactants, catalyst, and the ligand used in the alkyne coupling step. For example, suitable solvents include toluene, xylene (*ortho*-xylene, *meta*-xylene, *para*-xylene or mixtures thereof), benzene, water, methanol, ethanol, 1-propanol, 2-propanol, *n*-butanol, 2-butanol, pentanol, hexanol, *tert*-butanol, *tert*-amyl alcohol, ethylene glycol, 1,2-propanediol, 1,3-propanediol, glycerol, *N*-methyl-2-pyrrolidone, acetonitrile, *N,N*-dimethylformamide, methyl acetate, ethyl acetate, propyl acetate, isopropyl acetate, triacetin, acetone, methyl ethyl ketone, and ethereal solvents, such as 1,4-dioxane, tetrahydrofuran, 2-methyltetrahydrofuran, diethylether, cyclopenyl methyl ether, 2-butyl ethyl ether, dimethoxyethane, polyethyleneglycol. Mixtures of solvents may be suitably employed.

Optionally, water may be included in the fluorosulfonating step reaction mixture. One benefit of using fluorosulfonates as compared to triflates, is that the reaction can be carried out without a subsequent separation step, or with a simple separation step. In couplings involving triflates, a dedicated purification step is required to remove byproducts since the products and the byproducts typically occupy the same phase. In the reaction schemes described herein, the byproducts are either in the gas phase, and will bubble out spontaneously or with a simple degassing step, or will partition into the aqueous phase, which is easily separable. As such, the reaction scheme described herein provides additional benefits as compared to couplings involving triflates.

Following reaction with the fluorosulfonating agent, a poly(fluorosulfonate)-substituted aromatic compound is obtained. A fluorosulfonate substituent (or group) refers to -O-fluorosulfonate of the formula -OS(=O)₂F. Without being bound by theory, it is believed that the sulfur atom of the fluorosulfonate group is bonded to the oxygen of the hydroxyl groups of the polyhydroxy-substituted aromatic compound. At least two hydroxyl substituents of the polyhydroxyl-substituted aromatic compound are converted to fluorosulfonate substituents. The amount of the fluorosulfonating agent used is sufficient to convert at least two hydroxyl substituents on the aryl moiety of the aromatic compound to two fluorosulfonate substituents. If the polyhydroxy-substituted aromatic compound has more than two hydroxyl substituents on the aryl moiety, the amount of fluorosulfonate agent may vary from an amount sufficient to convert two of the hydroxyl substituents up to an amount sufficient to convert all of the hydroxyl substituents to fluorosulfonate substituents. The amount of fluorosulfonating agent used is typically greater than or equal to one equivalent for each hydroxyl substituent to be fluorosulfonated. Accordingly, the number of fluorosulfonate substituents desired in the poly(fluorosulfonate)-substituted aromatic compound depends on the desired number of alkyne moieties to be coupled to the aromatic compound. The fluorosulfonate substituents may be on the same aromatic ring or on different aromatic rings of the aryl moiety.

By way of example, Reaction Scheme 1 illustrates the general reaction of forming a poly(fluorosulfonate)-substituted aromatic compound of the formula (2) by reacting the polyhydroxy-substituted aromatic compound of formula (1) with a fluorosulfonating agent (FSA), where Ar, R¹, a, and b in both compounds (1) and (2) are as defined above for compound (1); c refers to the number of fluorosulfonate substituents and is an integer from 2 to 6; and d represents any remaining hydroxyl substituents and is an integer from 0 to 4. It will be appreciated by those skilled in the art that d = a - c in Reaction Scheme 1.

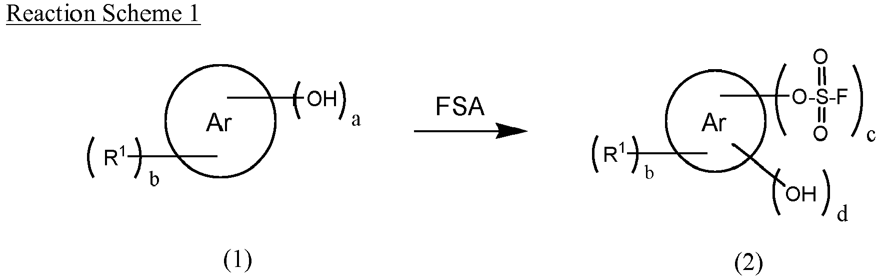

In the next step, the poly(fluorosulfonate)-substituted aromatic compound is reacted with an alkyne in the presence of a catalyst under conditions effective to couple the alkyne to the poly(fluorosulfonate)-substituted aromatic compound and form the desired polyalkynyl-substituted arene compound. The catalyst comprises one or more group 10 atoms or a copper complex. Optionally, one or more ligands, one or more bases, or a combination of one or more ligands and one or more bases may also be present in addition to the catalyst. This alkyne coupling reaction is typically performed in a solvent, and any of the solvents discussed above for the fluorosulfonating step are suitable for the alkyne coupling reaction. In this alkyne coupling reaction, the fluorosulfonate group serves as a leaving group from the aromatic compound. That is, a fluorosulfonate substituent in the aromatic compound is replaced with an alkyne moiety during the coupling reaction. The result of the coupling reaction is the formation of a new carbon-carbon bond between the aromatic compound and the alkyne. In general, this alkyne coupling step is illustrated by Reaction Scheme 2 where A represents an aromatic moiety and R-C≡C-H represents an alkyne.

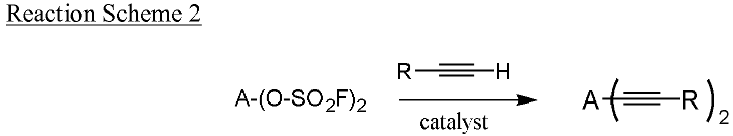

A wide variety of alkynes may be used in the coupling step to form the present polyalkynyl-substituted arenes. Such alkynes are terminal alkynes, that is, they have at least one carbon-carbon triple bond wherein one of the carbons that forms the carbon-carbon triple bonds is bonded to a hydrogen. Suitable alkynes may also have internal carbon-carbon triple bond linkages and/or carbon-carbon double bond linkages. Suitable alkynes may also contain one or more heteroatoms, such as nitrogen, oxygen, or sulfur. A typical alkyne useful in the present coupling reaction has the general formula R-C=C-H, where R is H or an organic moiety having from 1 to 30 carbon atoms. Such organic moiety may be aromatic or nonaromatic. Preferably, R is alkyl, heteroalkyl, hydroxyalkyl, C₆₋₃₀ aryl, C₆₋₃₀ aryl-substituted alkyl, -CO₂R^{a}, -Si(Z^{a})₃, or any other substituent compatible with a Sonogashira coupling; wherein R^{a} = H, alkyl, alkenyl, alkynyl, heteroalkyl, hydroxyalkyl, C₆₋₃₀ aryl, or C₆₋₃₀ aryl-substituted alkyl; and each Z^{a} is independently chosen from H, alkyl, heteroalkyl, C₆₋₃₀ aryl, C₆₋₃₀ aryl-substituted alkyl, and C₁₋₁₂ alkoxy. Preferred alkynes are those wherein R = C₁₋₁₀ alkyl, C₁₋₁₀ heteroalkyl, C₁₋₁₀ hydroxyalkyl, C₆₋₃₀ aryl, C₆₋₃₀ aryl-substituted alkyl,-CO₂R^{a}, or -Si(Z^{a})₃; wherein R^{a} = H, C₁₋₁₀ alkyl, C₂₋₁₀ alkenyl, C₂₋₁₀ alkynyl, C₁₋₁₀ heteroalkyl, C₁₋₁₀ hydroxyalkyl, C₆₋₃₀ aryl, C₆₋₃₀ aryl-substituted alkyl, or a C₁₋₃₀ hydrocarbyl moiety having one or more solubility enhancing substituents; and each Z^{a} is independently chosen from H, alkyl, C₆₋₃₀ aryl, C₆₋₃₀ aryl-substituted alkyl, and C₁₋₁₂ alkoxy. Suitable solubility enhancing substituents include, but are not limited to,-OH, C₁₋₁₂ hydroxyalkyl, -C(=O)OR¹³, -C(=O)N(R¹⁴)₂, -O-C(=O)R¹⁵, -NR¹⁴C(=O)R¹⁶, -NO₂; -S(=O)₂-OR¹⁷, -O-S(=O)₂-R¹⁸, -NR¹⁴-S(=O)₂-R¹⁶, S(=O)₂-N(R¹⁴)₂, and -Si(Z¹)₃; wherein R¹³ = H, C₁₋₁₂ alkyl, C₁₋₁₂ hydroxyalkyl, C₁₋₁₂ aminoalkyl, or C₆₋₃₀ aryl; each R¹⁴ is independently H, C₆₋₃₀ aryl, or C₁₋₁₂ alkyl; each R¹⁵ is independently chosen from H, C₁₋₁₂ alkyl, C₁₋₁₂ hydroxyalkyl, C₆₋₃₀ aryl, -O(C₁₋₁₂ alkyl), - O(C₆₋₃₀ aryl) and -N(R¹⁴)₂; R¹⁶ = H, C₁₋₁₂ alkyl, C₁₋₁₂ hydroxyalkyl, C₆₋₃₀ aryl, -O(C₁₋₁₂ alkyl), or -NH(C₁₋₁₂ alkyl); R¹⁷ = H, C₁₋₁₂ alkyl, or C₆₋₃₀ aryl; R¹⁸ = C₆₋₃₀ aryl, C₁₋₁₂ alkyl, and halo C₁₋₁₂ alkyl; each Z¹ is independently chosen from C₁₋₁₂ alkyl, C₁₋₁₂ hydroxyalkyl, C₁₋₁₂ alkoxy, C₁₋₁₂ alkanecarboxylate, -N(R¹⁴)₂, or -OSi(Y¹)₃; and each Y¹ is chosen from C₁₋₁₂ alkyl, C₁₋₁₂ hydroxyalkyl, C₁₋₁₂ alkoxy, or C₁₋₁₂ alkanecarboxylate. It will be appreciated by those skilled in the art that a mixture of alkynes may be used. It will also be apparent to those skilled in the art that any reactive functional groups, such as hydroxyl and carboxylic acid, in the terminal alkyne may be protected. Such hydroxyl and carboxylic acid protecting groups are well known to those skilled in the art. Suitable alkynes are generally commercially available, such as from Sigma-Aldrich, or may be prepared by methods known in the art.

Reaction Scheme 3 illustrates a preferred coupling reaction where poly(fluorosulfonate)-substituted aromatic compound of formula (2) is reacted with an alkyne (R-C=C-H) in the presence of a catalyst to provide a polyalkyne-substituted arene of formula (3), wherein Ar, R¹, R, a, b, c and d are as described above.

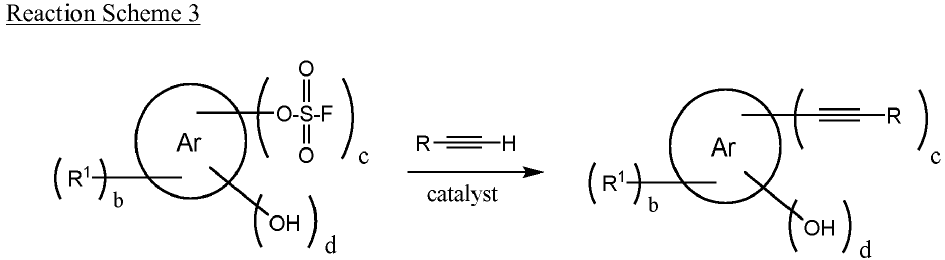

The alkyne coupling reaction herein uses one or more catalysts chosen from catalysts having at least one group 10 atom or a copper complex. A mixture of catalysts may be used, such as a mixture of a catalyst having at least one group 10 atom and a copper complex. Optionally, the alkyne coupling reaction may include a ligand and a base, in addition to the catalyst. The group 10 atoms include nickel, palladium and platinum. The catalyst is provided in a form suitable to the reaction conditions. For example, the catalyst may be provided on a substrate, or the catalyst having at least one group 10 atom may be generated *in situ* from one or more pre-catalysts and one or more ligands. In one instance, nickel-based catalysts are used. In another instance, platinum-based catalysts are used. In yet another instance, a catalyst including one or more of nickel, platinum and palladium-based catalysts are used.

Examples of palladium precatalysts include, but are not limited to, palladium(II) acetate, palladium(II) chloride, dichlorobis(acetonitrile)palladium(II), dichlorobis(benzonitrile)palladium(II), allylpalladium chloride dimer, palladium(II) acetylacetonate, palladium(II) bromide, bis(dibenzylideneacetone)palladium(0), bis(2-methylallyl)palladium chloride dimer, crotylpalladium chloride dimer, dichloro(1,5-cyclooctadiene)palladium(II), dichloro(norbornadiene)palladium(II), palladium(II) trifluoroacetate, palladium(II) benzoate, palladium(II) trimethylacetate, palladium(II) oxide, palladium(II) cyanide, tris(dibenzylideneacetone)dipalladium(0), palladium(II) hexafluoroacetylacetonate, cis-dichloro(N,N,N',N'-tetramethylethylenediamine)palladium(II), and cyclopentadienyl[(1,2,3-n)-1-phenyl-2-propenyl]palladium(II). In one instance, pyridine-enhanced precatalyst preparation stabilization and initiation (PEPPSI) type catalysts are used, for example, [1,3-bis(2,6-diisopropylphenyl)imidazol-2-ylidene](3-chloropyridyl)palladium(II) dichloride, and (1,3-bis(2,6-diisopropylphenyl)imidazolidene) ( 3-chloropyridyl) palladium(II) dichloride.

Examples of nickel precatalysts include, but are not limited to: nickel(II) acetate; nickel(II) chloride; bis(triphenylphosphine)nickel(II) dichloride; bis(tricyclohexylphosphine) nickel(II) dichloride; [1,1'-bis(diphenylphosphino)ferrocene]dichloronickel(II); dichloro[1,2-bis(diethylphosphino)ethane]nickel(II); chloro(1-naphthyl)bis(triphenylphosphine)nickel(II); 1,3-bis(2,6-diisopropylphenyl)imidazolium chloride; bis(1,5-cyclooctadiene)nickel(0); nickel(II) chloride ethylene glycol dimethyl ether complex; [1,3-bis(diphenylphosphino) propane]dichloronickel(II); [1,2-bis(diphenylphosphino)ethane]dichloronickel(II); and bis(tricyclohexylphosphine)nickel(0).

Ligands used in the alkyne coupling reaction mixture are preferably selected to generate the selected catalyst from a pre-catalyst. For example, the ligand may be a phosphine ligand, a carbene ligand, an amine-based ligand, a carboxylate-based ligand, an aminodextran, an aminophosphine-based ligands or an N-heterocyclic carbene-based ligand. In one instance, the ligand is monodentate. In another instance, the ligand is bidentate. In a further instance, the ligand is polydentate.

Suitable phosphine ligands may include, but are not limited to, mono- and bidentate phosphines containing functionalized aryl or alkyl substituents or their salts. For example, suitable phosphine ligands include, but are not limited to, triphenylphosphine; tri(o-tolyl)phosphine; tris(4-methoxyphenyl)phosphine; tris(pentafluorophenyl)phosphine; tri(p-tolyl)phosphine; tri(2-furyl)phosphine; tris(4-chlorophenyl)phosphine; di(1-adamantyl)(1-naphthoyl)phosphine; benzyldiphenylphosphine; 1,1'-bis(di-t-butylphosphino)ferrocene; (-)-1,2-Bis((2R,5R)-2,5-dimethylphospholano)benzene; (-)-2,3-bis[(2R,5R)-2,5-dimethylphospholanyl]-1-[3,5-bis(trifluoromethyl)phenyl]-1H-pyrrole-2,5-dione; 1,2-bis(diphenylphosphino)benzene; 2,2'-bis(diphenylphosphino)-1,1 '-binaphthyl; 2,2'-bis(diphenylphosphino)-1,1 '-biphenyl, 1,4-bis(diphenylphosphino)butane; 1,2-bis(diphenylphosphino)ethane; 2-[bis(diphenylphosphino)methyl]pyridine; 1,5-bis(diphenylphosphino)pentane; 1,3-bis(diphenylphosphino)propane; 1,1'-bis(di-i-propylphosphino)ferrocene; (S)-(-)-5,5'-bis[di(3,5-xylyl)phosphino]-4,4'-bi-1,3-benzodioxole; tricyclohexylphosphine(referred to herein as PCy3); tricyclohexylphosphine tetrafluoroborate (referred to herein as PCy3•HBF₄); N-[2-(di-1-adamantylphosphino)phenyl]morpholine; 2-(di-t-butylphosphino)biphenyl; 2-(di-t-butylphosphino)-3,6-dimethoxy-2',4',6'-tri-i-propyl-1,1'-biphenyl; 2-di-t-butylphosphino-2'-(N,N-dimethylamino)biphenyl; 2-di-t-butylphosphino-2'-methylbiphenyl; dicyclohexylphenylphosphine; 2-(dicyclohexylphosphino)-3,6-dimethoxy-2',4',6'-tri-i-propyl-1; 2-(dicyclohexylphosphino)-2'-(N,N-dimethylamino)biphenyl; 2-dicyclohexylphosphino-2',6'-dimethylamino-1,1'-biphenyl; 2-dicyclohexylphosphino-2',6'-di-i-propoxy-1,1'-biphenyl; 2-dicyclohexylphosphino-2'-methylbiphenyl; 2-[2-(dicyclohexylphosphino)phenyl]-1-methyl-1H-indole; 2-(dicyclohexylphosphino)-2',4',6'-tri-i-propyl-1,1'-biphenyl; [4-(N,N-dimethylamino) phenyl]di-t-butylphosphine; 9,9-dimethyl-4,5-bis(diphenylphosphino)xanthene; (R)-(-)-1-[(S)-2-(diphenylphosphino)ferrocenyl]ethyldicyclohexylphosphine; tribenzylphosphine; tri-t-butylphosphine; tri-n-butylphosphine; and 1,1'-bis(diphenylphosphino)ferrocene.

Suitable amine and aminophosphine-based ligands include any combination of monodentate or bidentate alkyl and aromatic amines including, but not limited to: pyridine; 2,2'-bipyridyl; 4,4'-dimethyl-2,2'-dipyridyl; 1,10-phenanthroline; 3,4,7,8-tetramethyl-1,10-phenanthroline; 4,7-dimethoxy-1,10-phenanthroline; N,N,N',N'-tetramethylethylenediamine; 1,3-diaminopropane; ammonia; 4-(aminomethyl)pyridine; (1*R*,2*S*,9*S*)-(+)-11-methyl-7,11-diazatricyclo[7.3.1.0^{2,7}]tridecane; 2,6-di-*tert*-butylpyridine; 2,2'-bis[(4*S*)-4-benzyl-2-oxazoline]; 2,2-bis((4*S*)-(-)-4-isopropyloxazoline)propane; 2,2'-methylenebis[(4*S*)-4-phenyl-2-oxazoline]; and 4,4'-di-tert-butyl-2,2'bipyridyl. In addition, aminophosphine ligands such as 2-(diphenylphosphino)ethylamine, 2-(2-(diphenylphosphino)ethyl)pyridine, (1R,2R)-2-(diphenylphosphino)cyclohexanamine, and 2-(di-*tert*-butylphosphino)ethylamine.

Suitable carbene ligands include N-heterocyclic carbene (NHC) based ligands, including, but not limited to: 1,3-bis(2,4,6-trimethylphenyl)imidazolinium chloride; 1,3-bis(2,6-diisopropylphenyl)imidazolium chloride; 1,3-bis-(2,6-diisopropylphenyl) imidazolinium chloride; 1,3-diisopropylimidazolium chloride, and 1,3-dicyclohexylbenzimidazolium chloride.

Optionally, a co-catalyst may be used in the present reaction in addition to the group 10 catalyst. In one instance, the co-catalyst is a copper complex as is known for use in Sonogashira couplings. In one instance, the copper complex is a halide salt of copper(I), for example, copper iodide or copper chloride. Without being limited by theory, it is possible that the use of both a group 10 catalyst and a copper catalyst serves to increase the rate of the reaction. Alternatively, a copper complex may be used as the catalyst, with or without the use of a group 10 catalyst.

The present polyalkynyl-substituted arenes may be prepared according to the present invention by performing discrete steps or by performing a one-pot reaction. When performed in discrete steps, the first step comprises reacting a polyhydroxy-substituted aromatic compound with a fluorosulfonating agent in the presence of a base to provide a poly(fluorosulfonate)-substituted aromatic compound, and the second step comprises coupling the poly(fluorosulfonate)-substituted aromatic compound with an alkyne in the presence of a catalyst. The crude polyfluorosulfonylated product does not need to be isolated, but instead can be directly carried forward in a single pot to undergo catalyzed Sonogashira cross-coupling. Accordingly, the present process may be performed as a one-pot reaction, the polyhydroxy-substituted aromatic compound is first reacted with a fluorosulfonating agent in the presence of a base and is then reacted with an alkyne in the presence of a catalyst.

Exemplary polyalkynyl-substituted arenes that can be prepared according to the present invention include, but are not limited to, the following structures, wherein each R is as defined above and each R⁹ is independently chosen from H and R¹, wherein each R¹ is as defined above.

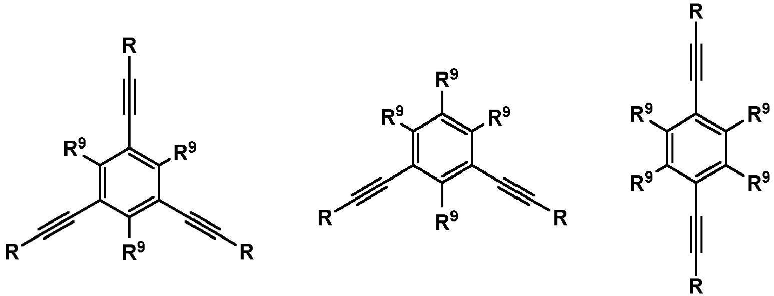

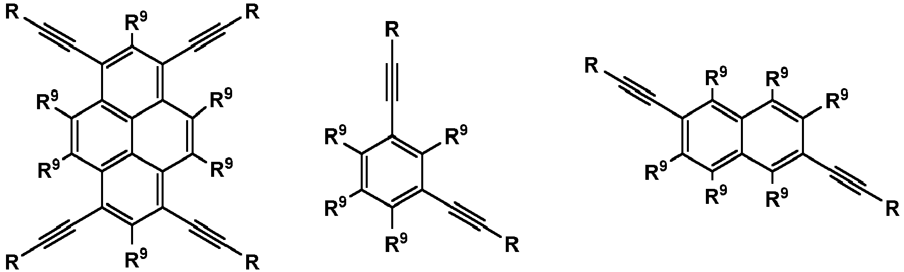

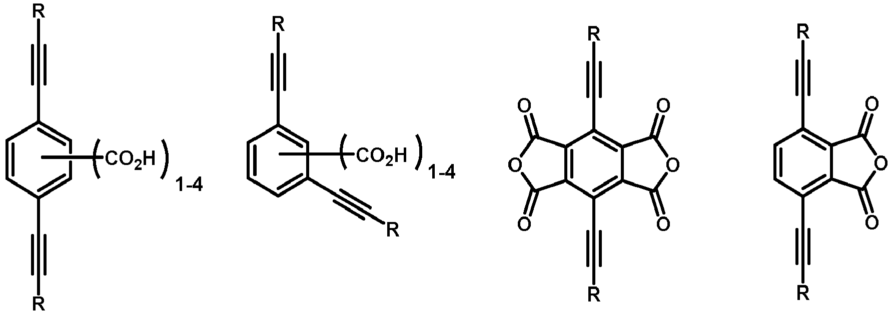

Example 1. To a 500 mL 3-necked, round bottom flask vented to a knockout pot and caustic scrubber is added phloroglucinol (10.0 g; 79.3 mmol) and potassium carbonate (36.3 g; 238 mmol). To the mixture is added 120 mL dimethylformamide (DMF). Sulfuryl fluoride is slowly bubbled sub-surface to the reaction mixture. The reaction mixture is purged with nitrogen and the knockout pot is disconnected and replaced with a nitrogen bubbler. To the reaction mixture are added 2-methyl-3-butyn-2-ol (19.2 mL; 208 mmol) and triethylamine (33.1 mL; 236 mmol) via syringe addition. Cyclopentadienyl[(1,2,3-n)-1-phenyl-2- propenyl]palladium(II) ("CpPd (cinnamyl)") (0.227 g; 0.79 mmol), triphenylphosphine (0.625 g; 2.36 mmol) and copper(I) iodide (0.605 g) are pre-weighted in a glovebox and the mixture is added to the reaction flask under slight positive nitrogen pressure. The reaction mixture is heated to 50 °C and is allowed to stir at this temperature until analysis of the mixture by liquid chromatography-mass spectrometry (LCMS) shows formation of the desired product. The crude reaction mixture is washed dilute HCl and extracted into 1 L of ethyl acetate. The volatiles are removed by rotary evaporation at 60 °C. The crude material is treated with 2 N aqueous sodium hydroxide (400 mL) and MTBE (400 mL) and the resulting biphasic mixture is transferred to a separatory funnel. The layers are shaken and separated. The aqueous layer is filtered through a bed of water-wet Celite and the cake is washed with 400 mL of water. With vigorous stirring, the filtrate is treated with 12.1 N hydrochloric acid until a pH <1 is obtained. The aqueous phase is transferred to a 2 L separatory funnel and extracted with methyl tert-butyl ether (MTBE) (2 x 200 mL). The combined organic extracts are evaporated to dryness. The solid residue is re-dissolved in diethyl ether (∼250 mL) and filtered through Celite. The filtrate is concentrated by rotary evaporation and then under high vacuum (<1 mmHg) at 50 °C and is expected to provide Compound 4.

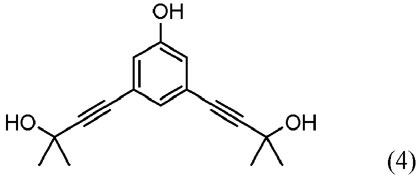

Example 2. A 30-mL glass scintillation vial fitted with a magnetic stir bar and a threaded cap is charged sequentially with Compound 4 from Example 1 (0.5 g, 1.93 mmol, 1.0 equiv.), powdered potassium hydroxide (0.572 g, 14.32 mmol, 7.4 equiv.), and 1,4-dioxane (10.0 mL). The vial is capped and placed in an aluminum block pre-heated to 100 °C and the reaction mixture is allowed to stir at this temperature for 120 min. The reaction mixture is then allowed to cool to room temperature and is diluted with 10 mL of 6 N hydrochloric acid and 75 mL of MTBE. The layers are shaken and allowed to separate. Solids that are present are removed by gravity filtration directly into a separatory funnel. The layers are shaken and separated and the organic phase is washed sequentially with DI water (1X) and brine (1X). The organic layer is decanted into a tared round-bottom flask and concentrated by rotary evaporation and then under high vacuum (<1 mmHg) and is expected to provide crude Compound 5.

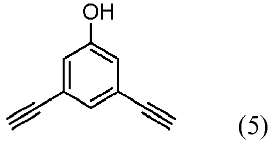

Example 3. To a 500 mL 3-necked, round bottom flask vented to a knockout pot and caustic scrubber was added 3,5-dihydroxybenzoic acid (10.0 g; 83.9 mmol) and potassium carbonate (38.4 g; 252 mmol). To the mixture was added 125 mL dimethylformamide (DMF). Sulfuryl fluoride was slowly bubbled sub-surface to the reaction mixture. After 6 hr., analysis by liquid chromatography -mass sprectrometry (LCMS) showed conversion of starting material to the desired bis-fluorosulfonate. The reaction mixture was purged with nitrogen and the knockout pot was disconnected and replaced with a nitrogen bubbler. To the reaction mixture was added 2-methyl-3-butyn-2-ol (20.3 mL; 220 mmol) and triethylamine (35 mL; 250 mmol) via syringe addition. CpPd(cinnamyl) (0.240 g; 0.84 mmol), triphenylphosphine (0.661 g; 2.52 mmol) and copper(I) iodide (0.640 g) were pre-weighted in a glovebox and the mixture was added to the reaction flask under slight positive nitrogen pressure. The reaction mixture turned a dark black color. The reaction mixture was heated to 50 °C overnight (15 hr.). LCMS analysis showed formation of the desired product and the mono-substituted intermediate in an approximate 2:1 ratio. After heating for an additional 6 hr., no change in the ratio of products was observed. To the mixture was added additional catalyst precursors CpPd(cinnamyl) (0.121 g), triphenylphosphine (0.330 g) and copper(I) iodide (0.320 g) that was pre-weighted in a glovebox. The reaction mixture was allowed to stir overnight at 50 °C. LCMS showed formation of the desired product. The crude reaction mixture was washed dilute HCl and extracted into 1 L of ethyl acetate. The volatiles were removed by rotary evaporation at 60 °C to reveal a black sludge. Analysis of the resulting material by ¹H NMR spectroscopy showed the desired product and DMF. The crude material was treated with 2 N aqueous sodium hydroxide (400 mL) and MTBE (400 mL) and the resulting biphasic mixture was transferred to a 2-L separatory funnel. The layers were shaken and separated. The aqueous layer was filtered through a bed of water-wet Celite and the cake was washed with approximately 400 mL of water. With vigorous stirring, the filtrate was treated with 12.1 N hydrochloric acid until a pH <1 was obtained. The pH swing induced the formation of cloudiness/solids. The heterogeneous aqueous phase was transferred to a 2 L separatory funnel and extracted with methyl tert-butyl ether (MTBE) (2 x 200 mL). The combined organic extracts were evaporated to dryness to provide a dark brown oily foam. The ¹H NMR spectrum of this material was collected in THF-d₈. The solid residue was re-dissolved in diethyl ether (∼250 mL) and filtered through Celite. The filtrate was concentrated by rotary evaporation and then under high vacuum (<1 mmHg) at 50 °C to provide Compound 6 as a dark brown oily foam (14.3 g; 59%). The compound was confirmed by ¹H and ¹³C NMR spectroscopy.

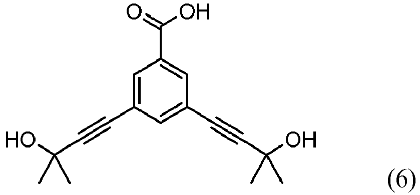

Example 4: A 30-mL glass scintillation vial fitted with a magnetic stir bar and a threaded cap was charged sequentially with Compound 6 from Example 3 (0.493 g, 0.84 mmol, 1.0 equiv., 49 wt% purity), powdered potassium hydroxide (0.399 g, 6.26 mmol, 7.4 equiv.), and 1,4-dioxane (5.0 mL). The vial was capped and placed in an aluminum block that had been pre-heated to 100 °C and the reaction mixture was allowed to stir at this temperature for 60 min. The reaction mixture was then allowed to cool slightly and then an approximate 0.1 mL sample was collected. The sample was diluted with 6 N hydrochloric acid and MTBE. The layers were shaken and allowed to separate. The aqueous (bottom) layer was removed and discarded. The organic layer was concentrated by rotary evaporation. The dark brown oily residue was dissolved in THF-*d₈* and analyzed by ¹H NMR spectroscopy. The reaction mixture was re-heated to 100 °C. After having been stirred for an additional 60 min., the reaction mixture was cooled and sampled. The sample was subjected to work-up and analysis as described above. The reaction mixture was diluted with 6 N hydrochloric acid (∼35 mL) and MTBE. Most of the insoluble dark brown solid material that was present was removed by gravity filtration directly into a separatory funnel. The layers were shaken and separated and the organic phase was washed sequentially with DI water (1X) and brine (1X). The organic layer was decanted into a tared round-bottom flask and concentrated by rotary evaporation and then under high vacuum (<1 mmHg) to leave crude 3,5-diethynylbenzoic acid (Compound 7) as an orange solid residue (0.223 g). A sample of the crude material (27.6 mg) was combined with 1,4-bis(trimethylsilyl)benzene (31.5 mg, 0.1412 mmol, 99.7 wt% purity) and the mixture was dissolved in THF-*d₈* (0.75 mL). The resulting solution was passed through a 0.2 µm nylon syringe filter and the filtrate was analyzed by ¹H NMR spectroscopy (16 scans, 20 second relaxation delay). On the basis of relative integration against the resonances of 1,4-bis(trimethylsilyl)benzene, the purity of Compound 7 was determined to be 28 wt%, which corresponded to a yield of 43%. The structure of the compound was confirmed by ¹H and ¹³C NMR spectroscopy.

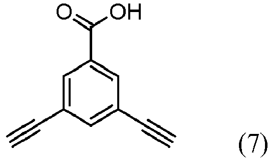

Example 5. To a 500 mL 3-necked, round bottom flask vented to a knockout pot and caustic scrubber is added dihydroxyisobenzofurandione (10.0 g; 55.5 mmol) and potassium carbonate (25.4 g; 167 mmol). To the mixture is added 90 mL dimethylformamide (DMF). Sulfuryl fluoride is slowly bubbled sub-surface to the reaction mixture. The reaction mixture is purged with nitrogen and the knockout pot is disconnected and replaced with an nitrogen bubbler. To the reaction mixture are added 2-methyl-3-butyn-2-ol (13.4 mL; 146 mmol) and triethylamine (23.2 mL; 165 mmol) via syringe addition. CpPd(cinnamyl) (0.159 g; 0.55 mmol), triphenylphosphine (0.438 g; 1.65 mmol) and copper(I) iodide (0.423 g) are pre-weighted in a glovebox and the mixture is added to the reaction flask under slight positive nitrogen pressure. The reaction mixture is heated to 50 °C and is allowed to stir at this temperature until LCMS shows formation of the desired product. The crude reaction mixture is washed dilute HCl and extracted into 1 L of ethyl acetate. The volatiles are removed by rotary evaporation at 60 °C. The crude material is treated with 2 N aqueous sodium hydroxide (325 mL) and MTBE (325 mL) and the resulting biphasic mixture is transferred to a separatory funnel. The layers are shaken and separated. The aqueous layer is filtered through a bed of water-wet Celite and the cake is washed with 400 mL of water. With vigorous stirring, the filtrate is treated with 12.1 N hydrochloric acid until a pH <1 is obtained. The aqueous phase is transferred to a 2 L separatory funnel and extracted with methyl tert-butyl ether (MTBE) (2 x 150 mL). The combined organic extracts are evaporated to dryness. The solid residue is re-dissolved in diethyl ether (∼200 mL) and filtered through Celite. The filtrate is concentrated by rotary evaporation and then under high vacuum (<1 mmHg) at 50 °C and is expected to provide Compound 8.

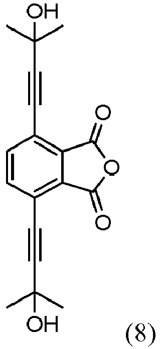

Example 6: A 30-mL glass scintillation vial fitted with a magnetic stir bar and a threaded cap is charged sequentially with Compound 8 from Example 5 (0.5 g, 1.60 mmol, 1.0 equiv.), powdered potassium hydroxide (0.474 g, 11.85 mmol, 7.4 equiv.), and 1,4-dioxane (10.0 mL). The vial is capped and placed in an aluminum block pre-heated to 100 °C and the reaction mixture is allowed to stir at this temperature for 120 min. The reaction mixture is then allowed to cool to room temperature and is diluted with 10 mL 6 N hydrochloric acid and 75 mL MTBE. The layers are shaken and allowed to separate. Gravity filtration into a separatory funnel is used to remove any solids that are present. The layers are shaken and separated and the organic phase is washed sequentially with DI water (1X) and brine (1X). The organic layer is decanted into a tared round-bottom flask and concentrated by rotary evaporation and then under high vacuum (<1 mmHg) and is expected to provide crude Compound 9.

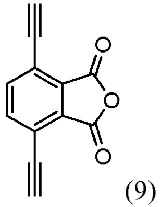

Example 7. The general procedure of Example 3 is repeated using the polyhydroxy-substituted aromatic compounds in Table 1 and the alkynes in Table 2 to prepare the polyalkynyl-substituted arene compounds in Table 3. In Table 3, the moles of fluorosulfonating agent (FSA) indicate the number of hydroxyl substituents converted to alkynyl substituents.

**Table 1**

| **Polyhydroxy Aromatic Compound** | **Chemical Name** |
|---|---|
| A | 1,1-Bi-2-naphthol |
| B | Bisphenol A |
| C | 1,3,5-Trihydroxybenzene |
| D | 2,3-Didihydroxynaphthalene |
| E | 2,3-Dihydroxypyridine |
| F | 2,5-Dihydroxyterephthalic acid |
| G | 2,6-Dihydroxytoluene |
| H | 2,4-Dihydroxypyrimidine-5-carboxylic acid |
| I | 4,8-dihydroxyquinoline-2-carboxylic acid |
| J | 1,6-dihydroxypyrene |
| K | 1,2-dihydroxyfluorene |

**Table 2**

| **Alkyne Compound** | **Chemical Name** |
|---|---|
| A | Ethynylbenzene |
| B | Propargyl alcohol |
| C | Propiolic acid |
| D | 2-Methyl-3-butyn-2-ol |
| E | 1-Butyne |
| F | 3-Ethynylpyridine |
| G | 1-Ethynyl-4-methoxybenzene |

**Table 3**

| **Polyalkynyl Arene** | **Polyhydroxy Aromatic Compound** | **Moles of FSA** | **Alkyne Compound** |
|---|---|---|---|
| 7-1 | A | 2 | A |
| 7-2 | C | 2 | D |
| 7-3 | C | 3 | A |
| 7-4 | E | 2 | G |
| 7-5 | D | 2 | C |
| 7-6 | I | 2 | E |
| 7-7 | H | 2 | A |
| 7-8 | J | 2 | C |
| 7-9 | K | 2 | D |
| 7-10 | F | 2 | A |
| 7-11 | G | 2 | F |
| 7-12 | B | 2 | B |
| 7-13 | H | 2 | F |

Example 8. A 3-neck 2-L round-bottom flask equipped with an overhead stirrer (PTFE paddle agitator), PTFE-coated thermocouple, sulfuryl fluoride (SO₂F₂) inlet line, and a Graham condenser fitted with a headspace outlet line that was vented to an aqueous sodium hydroxide (1 N) scrubber was charged with methyl 3,5-dihydroxybenozoate (30.17 g, 174 mmol, 1.0 equiv), powdered potassium carbonate (60.42 g, 433 mmol, 2.5 equiv) and 450 mL ethyl acetate (EtOAc). Overhead stirring was initiated and gaseous SO₂F₂ was bubbled subsurface into the reaction mixture for several hours. Once the reaction had reached full conversion (as evidenced by analysis of a filtered, concentrated reactor sample by ¹H NMR spectroscopy), the SO₂F₂ inlet line was replaced with a nitrogen inlet line and the system was purged for several minutes. The inorganic salts were removed by vacuum filtration through a 1 µm Whatman GFB glass microfiber medium that had been placed over a medium-porosity glass frit housing and the salt cake was washed with fresh EtOAc. The organic filtrate was concentrated by rotary evaporation and then under high vacuum (<1 mmHg) overnight to provide methyl 3,5-bis[(fluorosulfonyl)oxy]benzoate as a pale-yellow oil (57.62 g, 173 mmol, quantitative yield). The oil readily crystallized upon being transferred to a amber glass storage bottle. The product, methyl 3,5-bis[(fluorosulfonyl)oxy]benzoate, was confirmed by ¹H and ¹³C NMR spectroscopy.

Example 9. A 250-mL 3-neck round-bottom flask fitted with a PTFE-coated magnetic stir bar was charged with methyl 3,5-bis[(fluorosulfonyl)oxy]benzoate from Example 8 (5.00 g, 15.06 mmol, 1.0 equiv) and the flask was transferred to a nitrogen-filled glove box. In the nitrogen-filled glove box, a 10-mL glass scintillation vial was charged with triphenylphosphine (0.245 g, 0.92 mmol, 6 mol%), palladium(II) acetate (0.097 g, 0.43 mmol, 3 mol%) and copper(I) iodide (0.170 g, 0.89 mmol, 6 mol%). To the round-bottom flask containing methyl 3,5-bis[(fluorosulfonyl)oxy]benzoate was added 50 mL anhydrous dimethyl formamide (DMF) and, with magnetic stirring, the pre-catalyst mixture, which produced a dark tan-colored homogeneous solution. (Trimethylsilyl)acetylene (5.5 mL, 37.86 mmol, 2.5 equiv) and anhydrous triethylamine (5.3 mL, 37.84 mL, 2.5 equiv) were then added sequentially. The addition of (trimethylsilyl)acetylene was accompanied by a change in the reaction mixture color from dark tan to nearly black. The reaction flask was sealed with rubber septa, moved from the glove box to a fume hood, and fitted with a Dimroth condenser, nitrogen inlet, and a PTFE-coated thermocouple. At this point it became evident that the addition of triethylamine had induced an exotherm. Once the exotherm had peaked (ca. 45 °C), the reaction flask was fitted with a heating mantle and the reaction mixture was warmed to 50 °C and stirred at this temperature overnight. After having been stirred for 15 hours, analysis of the reaction mixture by GC/MS revealed that the starting material had been consumed and the desired product was cleanly formed. The reaction mixture was transferred to a single-neck round-bottom flask and concentrated by rotary evaporation (ca. 10 mmHg @ 60 °C). The resulting dark brown slurry was partitioned between MTBE (250 mL) and 1 N hydrochloric acid (100 mL), which induced the formation of a emulsion. The biphasic mixture was filtered through a bed of water-wet Celite^{®} and then the layers were separated. The rag layer that remained was taken with the aqueous layer. The organic layer was washed with 1 N hydrochloric acid (100 mL). The combined aqueous washes were extracted with MTBE (2 x 50 mL). The combined organic extracts were washed with half-saturated brine (100 mL), brine (1x), dried (anhydrous Na₂SO₄), filtered and concentrated by rotary evaporation. The resulting dark brown crude residue was dissolved in a minimum amount of 2% EtOAc/hexane and plug-filtered through a bed of silica gel (∼25 g) using additional 2% EtOAc/hexane as needed to completely elute the desired product. The filtrate was concentrated by rotary evaporation and then under high vacuum overnight (<1 mmHg) to afford methyl 3,5-bis[(trimethylsilyl)ethynyl]benzoate as a highly-viscous orange oil (4.61 g, 14.03 mmol, 93% yield). The oil crystallized upon standing at ambient temperature. The product, methyl 3,5-bis[(trimethylsilyl)ethynyl]benzoate, was confirmed by ¹H and ¹³C NMR spectroscopy.

Example 10. A 1-L 4-neck round-bottom flask fitted with an overhead stirrer (PTFE paddle agitator), nitrogen inlet, Dimroth condenser, 125-mL pressure-equalizing addition funnel and PFTE-coated thermocouple was charged with methyl 3,5-bis[(trimethylsilyl)ethynyl]benzoate (24.37 g, 74.17 mmol, 1.0 equiv) and methanol (300 mL). The reactor was sealed with a rubber septum and stirring was initiated. Gentle heating with a heat gun to ca. 35 °C was required for complete dissolution of the ester. Meanwhile, a sample of lithium hydroxide monohydrate (15.60 g, 372 mmol, 5.0 equiv) was combined with HPLC grade water (75 mL) and the resulting mixture was sonicated for several minutes to aid dissolution of the base. Any insoluble material that remained in the lithium hydroxide solution was removed by filtration and the filtrate was charged to the addition funnel. Aqueous lithium hydroxide solution was then added dropwise over the course of 5 minutes. During this time the internal temperature increased from 36 °C to 46 °C. After having been stirred for 1.5 hours at ambient temperature, a 0.1 mL sample of the reaction mixture was removed and diluted with 1 N HCl/CH₃CN (0.5 mL/4.0 mL). Analysis of the sample by LC/MS revealed that the desired product had been formed cleanly. The reaction mixture was transferred to a single-neck round-bottom flask and concentrated by rotary evaporation (∼10 mmHg @ 40 °C). The aqueous slurry was combined with HPLC grade water (300 mL) and MTBE (100 mL). The layers were shaken and separated and the organic layer was discarded as hazardous waste. The aqueous layer was transferred to a 1-L Erlenmeyer flask and, with vigorous stirring, the homogenous solution was acidified to a pH of approximately 1 using 6 N HCl (81 g), which induced the formation of a heavy cream-colored slurry. The slurry was combined with MTBE (150 mL) in a separatory funnel and the layers were shaken to produce a homogeneous two-phase system. The layers were separated and the aqueous layer was extracted with MTBE (2 x 150 mL). The combined organic extracts were stirred for several minutes with activated carbon (∼5 g, Darco^{®} G-60, 100 mesh) and then filtered. The filtrate was concentrated by rotary evaporation and then in a vacuum oven (<1 mmHg @ 50 °C) to provide 3,5-diethynylbenzoic acid as a cream-colored solid (11.56 g, 65.42 mmol, 88% yield). The product, 3,5-diethynylbenzoic acid, was confirmed by ¹H and ¹³C NMR spectroscopy. On the basis of quantitative ¹³C NMR spectroscopic analysis with pyrazine as an internal standard, the purity of 3,5-diethynylbenzoic acid was determined to be 96.3 wt%.

## Claims

1. A method of forming a polyalkynyl-arene compound comprising:
(a) reacting a polyhydroxy-substituted aromatic compound with a fluorosulfonating agent to form a poly(fluorosulfonate)-substituted aromatic compound; and
(b) reacting the poly(fluorosulfonate)-substituted aromatic compound with an alkyne in the presence of a catalyst under conditions effective to couple the alkyne to the poly(fluorosulfonate)-substituted aromatic compound and form the polyalkynyl-substituted arene compound;
wherein the catalyst comprises one or more group 10 atoms or a copper complex.

2. The method of claim 1 wherein the reaction mixture further comprises a ligand, and a base.

3. The method of claim 2 wherein the ligand includes one or more of a phosphine ligand, a carbene ligand, an amine-based ligand, and an aminophosphine-based ligand.

4. The method of claim 2 wherein the base is a carbonate salt, a phosphate salt, an acetate salt or a carboxylic acid salt.

5. The method of claim 1 wherein the aromatic compound has 1 to 8 aromatic rings.

6. The method of claim 1 wherein the aromatic compound has an aryl moiety derived from benzene, biphenyl, terphenyl, triphenylbenzene, hexaphenylbenzene, pyridine, bipyridine, pyrimidine, pyridazine, pyrrole, imidazole, triazine, triazole, furan, oxazole, thiazole, thiophene, fluorene, 9,9-diphenylfluorene, 9,9-dinaphthylfluorene, carbazole, dibenzofuran, dibenzothiophene, naphthalene, binaphthalene, phenanthracene, anthracene, pyrene, indole, isoindole, triphenylene, tetracene, tetraphene, pentacene, perylene, coronene, quinoline, isoquinoline, phthalic anhydride, pyromellitic dianhydride, benzimidazole, benzotriazole, benzofuran, benzothiophene, benzoxaxole, and benzothiazole.

7. The method of claim 1 wherein the aromatic compound is heteroaromatic.

8. The method of claim 1 wherein the aromatic compound has one or more substituents chosen from carboxylic acid, carboxylic acid anhydride, C₁₋₂₀ ester, substituted C₁₋₂₀ ester, C₁₋₁₂ alkoxy, C₁₋₁₂ alkyl, C₁₋₁₂ heteroalkyl, substituted C₁₋₁₂ alkyl, C₂₋₁₂ alkenyl, cyano, nitro, hydroxyl, protected hydroxyl, halo, amido, amino, mercapto, sulfido, silyl, phosphino, and phosphono.

9. The method of claim 1 wherein the catalyst is one or more of a palladium catalyst or a nickel catalyst or a copper complex.

10. The method of claim 1 wherein the alkyne is a primary alkyne.

11. The method of claim 1 wherein the catalyst comprises one or more group 10 atoms and a copper complex.

12. A method of forming a polyalkynyl-arene compound comprising:
(a) providing a poly(fluorosulfonate)-substituted aromatic compound; and
(b) reacting the poly(fluorosulfonate)-substituted aromatic compound with an alkyne in the presence of a catalyst under conditions effective to couple the alkyne to the poly(fluorosulfonate)-substituted aromatic compound and form the polyalkynyl-substituted arene compound;
wherein the catalyst comprises one or more group 10 atoms or a copper complex.
